# EUROPEAN PATENT APPLICATION

(11) **EP 4 332 984 A1**
(43) Date of publication of application: **06.03.2024**
(21) Application number: 22196074.3
(22) Date of filing: 16.09.2022
(51) Int. Cl.: G16H 30/40, G16H 40/20

(54) **SYSTEMS AND METHODS FOR IMPROVING COMMUNICATION BETWEEN LOCAL TECHNOLOGISTS WITHIN A RADIOLOGY OPERATIONS COMMAND CENTER (ROCC) FRAMEWORK**

(30) Priority: 31.08.2022 US 202263402489 P
(71) Applicant: Koninklijke Philips N.V., 5656 AG Eindhoven (NL)
(72) Inventor: STAROBINETS, Olga, Eindhoven (NL); CHADUVULA, Siva Chaitanya, Eindhoven (NL); KOKER, Ekin, 5656AG Eindhoven (NL); TELLIS, Ranjith Naveen, 5656AG Eindhoven (NL); DALAL, Sandeep Madhukar, Eindhoven (NL)
(74) Representative: Philips Intellectual Property & Standards

(57) **Abstract**

A non-transitory computer readable medium (**26s**) stores instructions executable by at least one electronic processor (**14s**) to perform a method (**100**) of providing assistance during a medical imaging examination performed using a medical imaging device (**2**). The method includes acquiring video and/or audio feeds (**17**, **18**) of two or more imaging examinations; identifying a feature of the two or more imaging examinations in one or more of the video and/or audio feeds; and establishing a natural communication pathway (**19**) between technicians (**LO**) performing the two or more imaging examinations based on the identified feature.

## Description

### FIELD OF THE INVENTION

The following relates generally to the imaging arts, remote imaging assistance arts, remote imaging examination monitoring arts, and related arts.

### BACKGROUND OF THE INVENTION

Medical imaging, such as computed tomography (CT) imaging, magnetic resonance imaging (MRI), positron emission tomography (PET) imaging, fluoroscopy imaging, and so forth, is a critical component of providing medical care, and is used in a wide range of medical fields, such as cardiology, oncology, neurology, orthopedics, to name a few. The operator of the medical imaging device used to acquire the medical images is typically a trained technologist, while interpretation of the medical images is often handled by a medical specialist such as a radiologist.

Currently, diagnostic imaging is in high demand. As the world population ages, the demand for quick, safe, high quality imaging will only continue to grow, putting further pressure on imaging centers and their staff. One approach for imaging centers to boost efficiency and grow operations without concomitant extra labor costs is through a radiology operations command center (ROCC) system. Radiology operations command centers enable remote experts to remotely provide assistance to imaging technicians at numerous imaging sites, providing their expertise as needed and remotely assisting less experienced technologists in carrying out high quality scans. Remote technologists or experts can monitor the local operators of scanning procedures through cameras installed in the scanning areas (or from other sources, such as sensors (including radar sensors), console video feeds, microphones connected to Internet of Things (IoT) devices, and so forth. In addition, these sources can be supplemented by other data sources like Health-Level 7 (HL7), Digital Imaging and Communications in Medicine (DICOM), Electronic Health Record (EHR) databases, and so forth.

The remote expert is often a senior imaging technologist (i.e. "super-tech") who, within the ROCC setup, is often concurrently assigned to assist a number of different imaging bays at different sites that may be spread out across different cities or different states. In practice, however, the super-tech can only be paying attention to a single imaging bay at any given time. The super-tech will typically be assisting local technologists who actively call for super-tech support.

ROCC systems are a promising way for large imaging centers or IDNs (integrated delivery networks) with diverse pools of senior technologist talent to remotely share their advice and expertise across the entire imaging network to imaging technicians who are actually performing imaging examinations. By providing means of communication and remote console sharing, ROCC aims to empower the most experienced technologists to provide guidance and oversight for junior techs when performing difficult scans or facing challenges during exam acquisition.

One of the main goals of ROCC is to empower the most experienced technologists within a large IDN to provide guidance and oversight for local junior technologists. Using tools built into the ROCC platform, remotely located expert users may virtually guide their less experienced colleagues through challenging acquisitions. However, exam acquisitions and exam problems happen in real time and may be difficult to predict, suggesting that availability of expert users may present a significant bottleneck.

The following discloses certain improvements to overcome these problems and others.

### SUMMARY OF THE INVENTION

In one aspect, a non-transitory computer readable medium stores instructions executable by at least one electronic processor to perform a method of providing assistance during a medical imaging examination performed using a medical imaging device. The method includes acquiring video and/or audio feeds of two or more imaging examinations; identifying a feature of the two or more imaging examinations in one or more of the video and/or audio feeds; and establishing a natural communication pathway between technicians performing the two or more imaging examinations based on the identified feature.

In another aspect, a non-transitory computer readable medium stores instructions executable by at least one electronic processor to perform a method of providing assistance during a medical imaging examination performed using a medical imaging device. The method includes acquiring video and/or audio feeds of two or more imaging examinations; identifying a location of each technician performing a corresponding imaging examination in one or more of the video and/or audio feeds; and establishing a natural communication pathway between technicians having a matching location.

In another aspect, a method of providing assistance during a medical imaging examination performed using a medical imaging device includes acquiring video and/or audio feeds of two or more imaging examinations; identifying a type of imaging examination for each imaging examination in one or more of the video and/or audio feeds; and establishing a natural communication pathway between technicians performing the two or more imaging examinations having a common identified type of imaging examination.

One advantage resides in providing tools for local imaging technicians to efficiently assist each other in various ways.

Another advantage resides in identifying imaging technicians performing examinations of the same type to establish communication pathways between such technicians, and providing tools for the identified imaging technicians to assist each other.

Another advantage resides in identifying imaging technicians performing examinations in a same location, to assist during such examinations.

Another advantage resides in identifying imaging technicians performing examinations in a same location to assist in emergency situations during such examinations.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically shows an illustrative apparatus for providing remote assistance in accordance with the present disclosure.
Fig. 2 shows an example flow chart of operations suitably performed by the apparatus of Fig. 1.

### DETAILED DESCRIPTION OF EMBODIMENT

While establishing open channels of communication between remotely located expert users and local technologists is important, it is recognized herein that the ROCC also provides an opportunity to streamline and strengthen communication and support among the local technologists. Sharing of knowledge through built-in ROCC features will create a more supportive environment for technologists, build expertise, and facilitate communication, ultimately resulting in better patient safety and improved exam quality.

The following is related to expanding the ROCC framework, developed to enable communication between a local imaging technologist performing an imaging examination and a remote expert, to also provide communication between local imaging technologists. As the local imaging technologists already have tablet computers or the like for communicating with remote experts, these tools can be modified to also facilitate targeted natural language communication between local imaging technologists.

In a first use case, the ROCC analyzes upcoming imaging examinations to identify pairs (or trios, or larger groups) of concurrent imaging examinations of the same type. When such overlap occurs, there is a reasonable likelihood that the imaging technicians performing these concurrent imaging examinations of the same type may be readily able to assist one another. This is because every imaging technician of the group has prepared to perform his or her assigned examination of that type, and so is inherently prepared to assist the others in the same type of imaging examination. Hence, at the time of the imaging examination each imaging technician of the pair or other group is notified of the other concurrent imaging examination(s) and provided videoconference links or the like to the other technicians. In the event that one imaging technician encounters a problem, rather than contacting a remote expert (who may be busy assisting with other imaging examinations), the imaging technician can contact one of the other imaging technicians identified by the ROCC as currently performing the same type of imaging examination, thus facilitating cooperative assistance between local imaging technicians.

The first use case can be applied across the entire ROCC, i.e. the paired imaging technicians could be located at different hospitals, or even in different states or countries. The next two use cases support cooperation between imaging technicians at the same location, where the proximity of the imaging technicians is leveraged to provide assistance in other ways.

The second use case recognizes that the imaging technician often has substantial periods of passive monitoring time. For example, while the imaging technician may be very busy during setup of imaging scans, the actual execution of the imaging scans is often fully automated, and at that stage of the examination the imaging technician is merely monitoring to ensure the scans are running properly and monitoring patient well-being. If the imaging technician wants to take a bathroom or rest break during such a passive monitoring period, he or she can contact an imaging technician in a neighboring imaging bay and ask whether that other imaging technician can briefly handle the passive monitoring during such a break. If accepted, the ROCC feeds from the imaging bay, such as the scraped screen and video camera feed, are transferred to the accepting imaging technician who can then briefly monitor the examination progress and patient well-being while the first imaging technician is on the break. To enable the second imaging technician to continue his or her primary examination, the transferred ROCC feeds can be shown in pop-up or picture-in-picture or split-screen windows on the tablet computer.

The third use case relates to an emergency situation. Here, if the ROCC cameras, microphones, or other sensors monitoring an imaging bay detects an emergency situation (such as a sudden collapse of the imaging technician or patient, an argument indicated by shouting, or so forth) in that imaging bay, then an alert is sent to the tablet computers of other local imaging technicians in neighboring imaging bays, possibly along with a pop-up window showing the video feed of the imaging bay experiencing the emergency, and/or a summary of the problem determined by artificial intelligence (AI) analysis of the sensor data. This enables rapid response to the emergency by the neighboring imaging technicians.

Yet another use case may involve a remotely located expert user on a call with the local technologist, observing a health episode involving the local technologist (seizure, stroke, heart attack, etc.). While the expert user may call emergency services, it would be prudent to also alert a healthcare professional in the vicinity, ensuring medical assistance may begin without delay. Ability to quickly identify and contact a technologist or technologist aid or a nurse within physical vicinity of a local technologist could be of benefit especially in emergency situations.

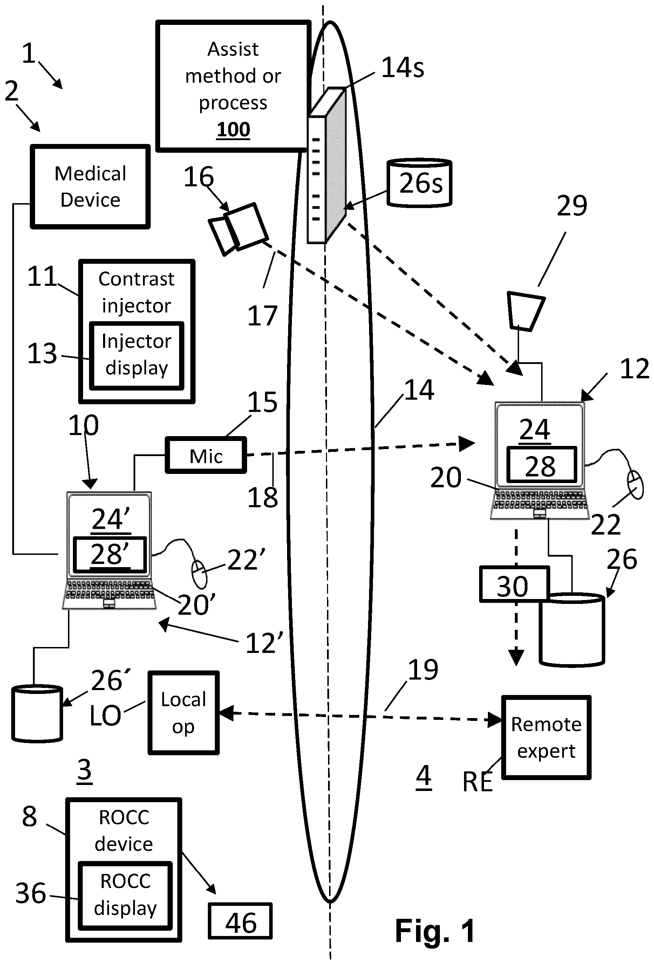

With reference to Fig. 1, an apparatus **1** for providing assistance from a remote medical imaging expert **RE** (or supertech) to a local technologist operator **LO** is shown. As shown in Fig. 1, the local operator **LO,** who operates a medical imaging device (also referred to as an image acquisition device, imaging device, and so forth) **2,** is located in a medical imaging device bay **3,** and the remote expert **RE** is disposed in a remote service location or center **4.** It should be noted that the "remote expert" **RE** may not necessarily directly operate the medical imaging device **2,** but rather provides assistance to the local operator **LO** in the form of advice, guidance, instructions, or the like. The remote location **4** can be a remote service center, a radiologist's office, a radiology department, and so forth. The remote location **4** may be in the same building as the medical imaging device bay **3** (this may , for example, in the case of a "remote operator or expert" **RE** who is a radiologist tasked with peri-examination image review), but more typically the remote service center **4** and the medical imaging device bay **3** are in different buildings, and indeed may be located in different cities, different countries, and/or different continents. In general, the remote location **4** is remote from the imaging device bay **3** in the sense that the remote expert **RE** cannot directly visually observe the imaging device **2** in the imaging device bay **3** (hence optionally providing a video feed as described further herein).

The image acquisition device **2** can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device **2** may also be a hybrid imaging device such as a PET/CT or SPECT/CT imaging system. While a single image acquisition device **2** is shown by way of illustration in Fig. 1, more typically a medical imaging laboratory will have multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, the remote service center **4** may provide service to multiple hospitals. The local operator controls the medical imaging device **2** via an imaging device controller **10.** The remote operator is stationed at a remote electronic processing device **12** (or, more generally, an electronic controller **12).**

Some types of imaging modalities and some types of imaging examinations may employ a contrast agent. For example, some types of MRI angiography imaging examinations employ a gadolinium-based magnetic contrast agent to observe blood flow into and out of an anatomical organ or region. To provide for such contrast-enhanced imaging, a programmable contrast injector **11** with a display **13** is configured to inject the patient with a contrast agent. In such an embodiment, the injector display **13** may be monitored by a further ROCC sensor such as a camera viewing the injector display **13.**

As used herein, the term "medical imaging device bay" (and variants thereof) refer to a room containing the medical imaging device **2** and also any adjacent control room containing the medical imaging device controller **10** for controlling the medical imaging device. For example, in reference to an MRI device, the medical imaging device bay **3** can include the radiofrequency (RF) shielded room containing the MRI device **2,** as well as an adjacent control room housing the medical imaging device controller **10,** as understood in the art of MRI devices and procedures. On the other hand, for other imaging modalities such as CT, the imaging device controller **10** may be located in the same room as the imaging device **2,** so that there is no adjacent control room and the medical bay 3 is only the room containing the medical imaging device **2.** In addition, while Fig. 1 shows a single medical imaging device bay **3,** it will be appreciated that the remote service center **4** (and more particularly the remote electronic processing device **12)** is in communication with multiple medical bays via a communication link **14,** which typically comprises the Internet augmented by local area networks at the remote expert **RE** and local operator **LO** ends for electronic data communications. In addition, while Fig. 1 shows a single remote service center **4,** it will be appreciated that the medical imaging device bays **3** is in communication with multiple medical bays via the communication link **14.**

As diagrammatically shown in Fig. 1, in some embodiments, a camera **16** (e.g., a video camera) is arranged to acquire a video stream or feed **17** of a portion of a workspace of the medical imaging device bay **3** that includes at least the area of the imaging device **2** where the local operator **LO** interacts with the patient, and optionally may further include the imaging device controller **10.** In other embodiments, a microphone **15** is arranged to acquire an audio stream or feed **18** of the workspace that includes audio noises occurring within the medical imaging device bay **3** (e.g., verbal instructions by the local operator **LO,** questions from the patient, and so forth). The video stream **17** and/or the audio stream **18** is sent to the remote electronic processing device **12** via the communication link **14,** e.g. as a streaming video feed received via a secure Internet link.

The communication link **14** also provides a natural language communication pathway **19** for verbal and/or textual communication between the local operator and the remote operator. For example, the natural language communication link **19** may be a Voice-Over-Internet-Protocol (VOIP) telephonic connection, an online video chat link, a computerized instant messaging service, or so forth. Alternatively, the natural language communication pathway **19** may be provided by a dedicated communication link that is separate from the communication link **14** providing the data communications **17, 18,** e.g. the natural language communication pathway **19** may be provided via a landline telephone. In some embodiments, the natural language communication link **19** allows a local operator **LO** to call a selected remote expert **RE.** The call, as used herein, can refer to an audio call (e.g., a telephone call), a video call (e.g., a Skype or Facetime or other screen-sharing program), or an audio-video call. In another example, the natural language communication pathway **19** may be provided via an ROCC device **8** with a display device **36.** For example, an "app" can run on the ROCC device **8** (operable by the local operator **LO)** and the remote electronic processing device **12** (operable by the remote expert **RE)** to allow communication (e.g., audio chats, video chats, and so forth) between the local operator and the remote expert.

Fig. 1 also shows, in the remote service center **4** including the remote electronic processing device **12,** such as a workstation, a workstation computer, or more generally a computer, which is operatively connected to receive and present the video feed **17** of the medical imaging device bay **3** from the camera **16** and/or to the audio feed **18.** Additionally or alternatively, the remote workstation **12** can be embodied as a server computer or a plurality of server computers, e.g. interconnected to form a server cluster, cloud computing resource, or so forth. The workstation **12** includes typical components, such as an electronic processor **20** (e.g., a microprocessor), at least one user input device (e.g., a mouse, a keyboard, a trackball, and/or the like) **22,** and at least one display device **24** (e.g. an LCD display, plasma display, cathode ray tube display, and/or so forth). In some embodiments, the display device **24** can be a separate component from the remote electronic processing device **12.** The display device **24** may also comprise two or more display devices. The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the workstation **12,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the remote operator display device **24.** The video feed **17** from the camera **16** can also be displayed on the display device **24,** and the audio feed **18** can be output on the remote electronic processing device **12** via a loudspeaker **29.** In some examples, the audio feed **18** can be an audio component of an audio/video feed (such as, for example, recording as a video cassette recorder (VCR) device would operate).

Fig. 1 shows an illustrative local operator **LO,** and an illustrative remote expert **RE** (e.g., supertech). However, in a Radiology Operations Command Center (ROCC) as contemplated herein, the ROCC provides a staff of supertechs who are available to assist local operators **LO** at different hospitals, radiology labs, or the like. Each remote expert **RE** can operate a corresponding remote electronic processing device **12.** The ROCC may be housed in a single physical location, or may be geographically distributed. For example, in one contemplated implementation, the remote expert **RE** are recruited from across the United States and/or internationally in order to provide a staff of supertechs with a wide range of expertise in various imaging modalities and in various imaging procedures targeting various imaged anatomies. A server computer **14s** can be in communication with the medical imaging bay **3** and the remote service center **4** with one or more non-transitory storage media **26s.** The non-transitory storage media **26s** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer **14s,** various combinations thereof, or so forth. It is to be understood that any reference to a non-transitory medium or media **26s** herein is to be broadly construed as encompassing a single medium or multiple media of the same or different types. Likewise, the server computer **14s** may be embodied as a single electronic processor or as two or more electronic processors. The non-transitory storage media **26s** stores instructions executable by the server computer **14s.**

The medical imaging device controller **10 in** the medical imaging device bay **3** also includes similar components as the remote electronic processing device **12** disposed in the remote service center **4.** Except as otherwise indicated herein, features of the medical imaging device controller **10,** which includes a local workstation **12',** disposed in the medical imaging device bay **3** similar to those of the remote workstation **12** disposed in the remote service center **4** have a common reference number followed by a "prime" symbol, and the description of the components of the medical imaging device controller **10** will not be repeated. In particular, the medical imaging device controller **10** is configured to display a GUI **28'** on a display device or controller display **24'** that presents information pertaining to the control of the medical imaging device **2,** such as configuration displays for adjusting configuration settings an alert **30** perceptible at the remote location when the status information on the medical imaging examination satisfies an alert criterion of the imaging device **2,** imaging acquisition monitoring information, presentation of acquired medical images, and so forth. It will be appreciated that the screen mirroring data stream **18** carries the content presented on the display device **24'** of the medical imaging device controller **10.** The communication link **14** allows for screen sharing between the display device **24** in the remote service center **4** and the display device **24'** in the medical imaging device bay **3.** The GUI **28'** includes one or more dialog screens, including, for example, an examination/scan selection dialog screen, a scan settings dialog screen, an acquisition monitoring dialog screen, among others. The GUI **28'** can be included in the video feed **17** and displayed on the remote workstation display **24** at the remote location **4.**

Furthermore, as disclosed herein, the server **14s** performs a method or process **100** for providing assistance during a medical imaging examination performed using a medical imaging device **2** (i.e., by assisting local operators **LO** of respective medical imaging devices **2** during medical imaging examinations by a remote expert **RE).** The method or process **100** is further operative to provide assistance between local operators **LO,** as disclosed herein. The instructions to perform the method **100** are stored in the non-transitory computer readable medium **26** of the remote workstation **12.**

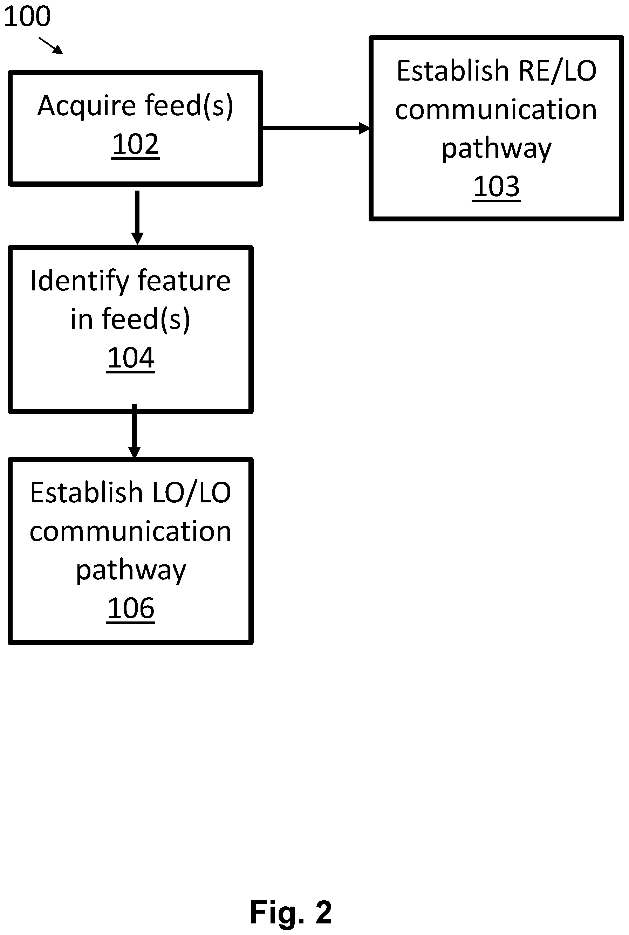

With reference to Fig. 2, and with continuing reference to Fig. 1, an illustrative embodiment of the method **100** is diagrammatically shown as a flowchart. To begin the method **100,** multiple (i.e., two or more imaging examinations are commenced by corresponding local operators **LO** using corresponding medical imaging devices **2** disposed in corresponding imaging bays **3.** The two or more imaging examinations can occur in a common medical facility, or in different medical facilities.

At an operation **102,** the video feed **17** (acquired by the one or more cameras **16** and/or the audio feed **18** (acquired by the one or more microphones **15)** of each imaging examination are acquired and routed to the server computer **14s** for analysis. This constitutes the data collection used for normal ROCC operations, that is, to provide the remote expert **RE** with information sufficient to enable the remote expert **RE** to assist a local operator **LO** during an imaging examination.

At an operation **103,** a communication pathway between a local operator **LO** and the remote expert **RE** is established to enable the latter to assist the former. The operation **103** may occur in response to the local operator **LO** making a request for remote expert assistance via the ROCC device **8,** and the communication pathway may, by way of nonlimiting illustrative example, comprise a telephonic or videoconference link established between the local operator **LO** and the assisting remote expert **RE,** along with sharing of the controller display and imaging bay sensor data with the remote expert **RE.**

As disclosed herein, however, the ROCC framework is also used in the method **100** to provide assistive communication between two (or more) local operators. Since there may be dozens, hundreds, or more local operators performing imaging examinations under ROCC monitoring at any given time, the method **100** promotes targeted local operator-local operator assistance by identifying local operators that are likely to be in a position to assist one another. To this end, at an operation **104,** a common feature of the two or more imaging examinations are identified in the video feed **17** and/or the audio feed **18.** At an operation **106,** the natural communication pathway **19** is established between local technicians **(LO)** performing the two or more imaging examinations based on the identified feature.

The feature identification operation **104** can be performed in a variety of manners. In some embodiments, the identified feature is a type of imaging examination of each imaging examination. For example, the type of imaging examination can be a modality of the imaging device **2,** an anatomy of the patient being imaged, or both. The natural communication pathway **19** can then be established between the local technicians **LO** performing the two or more imaging examinations having a common identified type of imaging examination. In some examples, an indication **46** of an identification of each local technician **LO** performing the common type of imaging examination can be displayed on the display device **36** on corresponding electronic processing devices **8** operable by each corresponding local technician **LO.** This approach for targeted assistance between local operators leverages the expectation that each of the local operators performing these concurrent imaging examinations have the training, and have prepared, to perform the imaging examination. Hence, at the time of the concurrent imaging examinations each of these identified local operators is notified of the other concurrent imaging examination(s) and provided videoconference links or the like to the other technicians. In the event that one local operator encounters a problem, he or she can contact one of the other imaging technicians identified by the ROCC as currently performing the same type of imaging examination, thus facilitating cooperative assistance between local imaging technicians. This has the substantial benefit that the valuable time of the remote expert **RE** is not occupied in resolving the problem. (Howbeit, if the cooperative efforts of the local operators is insufficient to resolve the problem, the local operator **LO** experiencing the problem can escalate by contacting the remote expert **RE** for assistance in accordance with the operation **103).**

In other embodiments, the feature that is identified in the operation **104** is a location of each imaging examination (i.e., at the same medical facility, or different medical facilities). The natural communication pathway **19** can then be established between the local technicians **LO** performing the two or more imaging examinations identified as being at matching locations. By "matching" it is meant that the locations are in the same radiology facility (e.g., in a single building, or in a single radiology suite within a single building), and/or a distance between the imaging examinations is at or below a threshold distance as measured by a suitable distance metric. Determining that two (or more) locations match can be done in various ways. In one approach, if the examinations are tagged as to radiology facility, then the matching locations can be recognized as having the same radiology facility tags. In another approach, if the electronic processing devices **8** used in the examinations have GPS and/or other geographical location services then this can be used to determine the distance. These are merely illustrative examples. In some examples, a time period during which each local technician **LO** in the matching location is passive (i.e., when the local technician **LO** is not actively performing a step or process in their respective imaging examination). A displayed indication **46** can be displayed on the display device **36** on corresponding electronic processing devices **8** operable by each corresponding local technician **LO** of each passive time period for each corresponding local technician **LO** in the matching location. One of the local technicians **LO** in the matching location can send messages to the other local technicians **LO** in the identified matching location, which can be displayed on the display device **36** on corresponding electronic processing devices **8** operable by each corresponding local technician **LO.** The messages can be, for example, a request for assistance, a request to monitor the sender's imaging examination while that local technician **LO** takes a break (i.e., restroom, phone call, lunch, and so forth). The "other" local technicians **LO** can provide user inputs to the corresponding electronic processing device **8** indicative of an acceptance or rejection of the message (i.e., "yes, I can assist with your imaging examination while you take a break", "no, I cannot assist you" and obvious variants thereof). If one of the local technicians **LO** agrees to assist the sender of the message (i.e., accepts the message), the video and/or audio feeds **17, 18** of the imaging examination performed by the technician who generated the message can be transferred to the corresponding electronic processing device **8** of the local technician **LO** who accepted the message. The display device **36** of the electronic processing device **8** of the local technician **LO** who accepted the message can display a first window the video and/or audio feeds **17, 18** of the imaging examination performed by the local technician **LO** who provided the user input (i.e., accepted the message), and a second window showing the video and/or audio feeds **17, 18** of the imaging examination performed by the local technician **LO** who generated the message (i.e., requested assistance).

In a further embodiment, the feature identified in the operation **104** can comprise an emergency condition occurring during one of the imaging examinations. In this case, imaging bay video feed(s) and/or data from other sensors monitoring the imaging bay are analyzed by artificial intelligence (AI) and/or other analysis to detect a possible emergency situation in the imaging bay. As one example, successive frames (or frames spaced apart in time by a chosen time interval) can be compared to detect a collapse of the patient or the local operator (suggestive of a possible heart attack or other acute medical problem). As another example, audio feed from an imaging bay microphone can be monitored to detect elevated volume potentially indicative of an argument, or more specific analysis can be done by AI to detect, specifically a raised voice. As yet another example, the audio feed can be monitored to detect usage of inappropriate language suggesting that intervention may be called for. Upon detection of an emergency condition, an alert indicative of the emergency condition is sent to the corresponding electronic processing devices **8** operable by each corresponding technician **LO** at the matching location. In some examples, the video feed **17** of the imaging examination in which the emergency condition is occurring can be displayed in each corresponding ROCC device **8.**

In the following, some further examples, embodiments, and variants are described. Quite a few studies have reported that the quality of teamwork in a medical setting is directly reflected in patient outcomes. In a fast-paced environment of radiology departments, effective teamwork is essential but can be difficult to manage. One way of teaming up could be to pair technologists performing the same type of scan in different locations across an IDN. A basic matching scheme could be applied to exam schedules, creating potential connections that local technologists may take advantage of at the time of acquisition. Such a system could reduce the demand for expert user intervention, improve protocol adherence, and reduce errors. Local technologists **LO** may connect through existing ROCC communication channels and exchange screenshots to compare acquisition parameters, image quality, patient positioning, etc. Additionally, if local technologists **LO** run into issues, they could invite the help from a remote expert **RE.** This way the remote expert **RE** could oversee and assist multiple ongoing exams. Performance of technologist pairs could be tracked and evaluated using standard KPIs such as number of repeated sequences, exam duration, exam quality, adherence to protocols, etc. This data could be used to identify the most optimal pairings, the exams for which pairing techs is particularly advantageous, etc. These insights could be further used for optimizing exam schedules across IDNs to maximize the overall performance of technologists, ensuring high exam quality and compliance.

Another opportunity for local technologists to work in tandem is to keep an eye on their neighbor's scan while he or she steps away from the imaging bay **3 -** either for lunch or to prep the following patient. In reality, most scans are rather straight forward. Once initial images are acquired, the rest of the exam sequences can be prescribed, and an auto-scan option can be activated. The role of the local technician **LO** switches from active (patient positioning and coaching, initial acquisitions and exam set-up) to passive (watching for motion artifact in acquired images and ensuring the patient remains comfortable). A neighboring local technician LO could perform this passive watch especially when provided with appropriate tools. Image quality during acquisition could be assessed 1) automatically by an image quality tracking algorithm that kicks in when a technologist leaves his station or 2) manually by a neighboring technologist who through ROCC receives images as they are being acquired at the neighboring scanner. If the image quality is poor, the responsible local technologist **LO** might be summoned back to his station, or an expert user may be asked to intervene. These summons and interventions by the remote expert **RE** will be tracked and are used to train models to identify exams that should not be left unattended, as well as exams that are not likely to require intervention. Such models will allow local technologists to better plan and balance current and upcoming exams. To ensure patient is comfortable, patient alarm button is going to be monitored (via camera) and status continuously displayed on the ROCC device **8.** If the patient squeezes the alarm ball, the message is displayed in the ROCC device **8,** neighbor tech, as well as the responsible tech are both alerted.

Rarely, emergency situations may arise that require hands-on help from staff members in the vicinity. Such situations may include patients experiencing serious medical problems (heart attacks, seizures, etc.), falls, or perhaps patients acting in a combative manner that alarms the technologist. Processing camera feed for audio and video ques indicative of such scenarios and then sending alarm could speed up the response. Local staff in the vicinity of the incident might be alerted that assistance is required. In one embodiment, a local technologist scanning at night by himself may run into problems if a patient has a medical episode. The apparatus **1** automatically call for help, leaving the local technologist **LO** to offer first aid to the patient.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A non-transitory computer readable medium **(26s)** storing instructions executable by at least one electronic processor **(14s)** to perform a method **(100)** of providing assistance during a medical imaging examination performed using a medical imaging device **(2),** the method comprising:
acquiring video and/or audio feeds (**17, 18**) of two or more imaging examinations;
identifying a feature of the two or more imaging examinations in one or more of the video and/or audio feeds; and
establishing a natural communication pathway **(19)** between technicians **(LO)** performing the two or more imaging examinations based on the identified feature.

2. The non-transitory computer readable medium **(26s)** of claim 1, wherein identifying a feature of the two or more imaging examinations in one or more of the video and/or audio feeds **(17. 18)** includes:
identifying a type of imaging examination for each imaging examination;
establishing the natural communication pathway **(19)** between technicians **(LO)** performing the two or more imaging examinations having a common identified type of imaging examination.

3. The non-transitory computer readable medium **(26s)** of claim 2, wherein the method **(100)** further includes:
displaying an indication **(46)** of an identification of each technician performing the common type of imaging examination, the indication being displayed on a corresponding electronic processing device **(8)** operable by each corresponding technician.

4. The non-transitory computer readable medium **(26s)** of any one of claims 1-3, wherein identifying a feature of the two or more imaging examinations in one or more of the video and/or audio feeds (**17, 18**) includes:
identifying a location of each technician **(LO)** performing a corresponding imaging examination
establishing the natural communication pathway **(19)** between technicians **(LO)** having a matching location.

5. The non-transitory computer readable medium **(26s)** of claim 4, wherein the method **(100)** further includes:
identifying a time period during which each technician **(LO)** in the matching location is passive; and
displaying an indication **(46)** of an identification of each passive time period for each corresponding local technician in the matching location, the indication being displayed on a corresponding electronic processing device **(8)** operable by each corresponding technician.

6. The non-transitory computer readable medium **(26s)** of claim 4, wherein the method **(100)** further includes:
receiving a message from one of the technicians **(LO)** in the matching location, the message being displayed on each electronic processing device **(8)** operable by each corresponding technician other than the technician who generated the message;
receiving, on one of the electronic processing devices operable by each corresponding technician other than the technician who generated the message, a user input indicative of an acceptance or rejection of the message.

7. The non-transitory computer readable medium **(26s)** of claim 6, wherein the message comprises a request for a break for the technician **(LO)** who generated the message to take a break from performing the corresponding imaging examination, and the method **(100)** further includes:
receiving, on one of the electronic processing devices operable by each corresponding technician other than the technician who generated the message, a user input from the corresponding technician indicative of an acceptance of the message; and
upon receiving the user input, transferring the video and/or audio feeds **(17, 18)** of the imaging examination performed by the technician who generated the message to the technician who provided the user input.

8. The non-transitory computer readable medium **(26s)** of claim **7,** wherein the method **(100)** further includes:
displaying, on the electronic processing device **(8)** operable by the technician **(LO)** who provided the user input indicative of an acceptance of the message, a first window showing the video and/or audio feeds (**17, 18**) of the imaging examination performed by the technician who provided the user input, and a second window showing the video and/or audio feeds of the imaging examination performed by the technician who generated the message.

9. The non-transitory computer readable medium **(26s)** of claim 4, wherein identifying a feature of the two or more imaging examinations in one or more of the video and/or audio feeds **(17. 18)** includes:
identifying an emergency condition occurring during one of the imaging examinations; and
sending an alert indicative of the emergency condition to the corresponding electronic processing device **(8)** operable by each corresponding technician.

10. The non-transitory computer readable medium **(26s)** of claim 9, wherein the method **(100)** further includes:
displaying, on each corresponding electronic processing device **(8)** operable by each corresponding technician, a video feed of the imaging examination in which the emergency condition is occurring.

11. The non-transitory computer readable medium **(26s)** of any one of claims 1-10, wherein the feed (**17, 18**) comprises a video feed acquired by a camera **(16)** disposed in a medical imaging bay **(3)** where the imaging examination is performed.

12. The non-transitory computer readable medium **(26s)** of any one of claims 1-10, wherein the feed (**17, 18**) comprises an audio feed acquired by a microphone **(15)** disposed in a medical imaging bay **(3)** where the imaging examination is performed.

13. A non-transitory computer readable medium **(26s)** storing instructions executable by at least one electronic processor **(14s)** to perform a method **(100)** of providing assistance during a medical imaging examination performed using a medical imaging device **(2),** the method comprising:
acquiring video and/or audio feeds (**17, 18**) of two or more imaging examinations;
identifying a location of each technician **(LO)** performing a corresponding imaging examination in one or more of the video and/or audio feeds; and
establishing a natural communication pathway **(19)** between technicians **(LO)** having a matching location.

14. The non-transitory computer readable medium **(26s)** of claim 13, wherein the method **(100)** further includes:
identifying a time period during which each technician **(LO)** in the matching location is passive; and
displaying an indication **(46)** of an identification of each passive time period for each corresponding local technician in the matching location, the indication being displayed on a corresponding electronic processing device **(8)** operable by each corresponding technician.

15. The non-transitory computer readable medium **(26s)** of claim 14, wherein the method **(100)** further includes:
receiving a message from one of the technicians **(LO)** in the matching location, the message being displayed on each electronic processing device **(8)** operable by each corresponding technician other than the technician who generated the message;
receiving, on one of the electronic processing devices operable by each corresponding technician other than the technician who generated the message, a user input indicative of an acceptance or rejection of the message.
